# EUROPEAN PATENT APPLICATION

(11) **EP 1 074 629 A1**
(43) Date of publication of application: **07.02.2001**
(21) Application number: 99202524.7
(22) Date of filing: 30.07.1999
(51) Int. Cl.: C12N 15/82, C12N 5/10, A01H 5/00

(54) **Sink protein**

(71) Applicant: Coöperatieve Verkoop- en Productievereniging van Aardappelmeel en Derivaten 'AVEBE' B.A., NL-9641 JA Veendam (NL)
(72) Inventor: The designation of the inventor has not yet been filed
(74) Representative: Ottevangers, Sietse Ulbe

(57) **Abstract**

The invention relates to the field of providing additional nutritional value to a (micro-)organism by equipping a cell or cells of said organism with means for producing desired levels of an amino acid, preferably a so-called essential amino acid.

The invention provides a method for regulating the flux, concentration or biosynthesis of a desired amino acid in a cell comprising providing said cell with a nucleic acid encoding a proteinaceous substance comprising at least said amino acid, such a proteinaceous substance herein also called sink protein whereby sink stands for the capacity of said proteinaceous substance to function as sink or reservoir for the desired amino acid, thereby regulating or reducing the amount or concentration of available of free amino acid in said cell by binding it through transcription and translation processes to the sink protein encoded by the additional nucleic acid.

## Description

The invention relates to the field of providing additional nutritional value to a (micro-)organism by equipping a cell or cells of said organism with means for producing desired levels of an amino acid, preferably a so-called essential amino acid.

Amino acids are the essential building blocks of proteins. Proteins (herein also meaning (poly)peptides) are in essence linear polymers of amino acids joined head to tail by an amide linkage, a peptide bond, between the carboxylic acid group of one amino acid and the amino group of the next amino acid. There are 20 common amino acids used for the biosynthesis of proteins, each with a different side chain attached to a central α-carbon atom. The same 20 amino acids occur over and over again in all proteins, including those made by lower organisms such as bacteria, yeast and fungi, and by higher organisms such as plants and animals. The chemical versatility provided by amino acids to proteins is determined by the properties of the amino acid side chains, the varied amino acid composition of proteins determines the properties of proteins and underlies all of the diverse and sophisticated functions of proteins.

Amino acids that are not utilised in biosynthesis of proteins can be used for great many other purposes by the cell. For example, they can be oxidised to generate metabolic energy. Most of their carbon and hydrogen atoms eventually form CO₂ or H₂O, while their nitrogen atoms are shuttled through various forms and eventually can appear as urea, which is excreted. Many amino acids play a central role in various crucial cellular pathways. A common amino acid such as serine, for example, can be modified chemically in different ways, it can be linked to AMP in preparation for protein synthesis, it can be degraded to glycine, converted to pyruvate in preparation for oxidation, acetylated by Acetyl CoA or transferred to a fatty acid to make phosphadityl serine.

Another amino acid, tryptophan is one of the least abundant yet, in terms of energy, one of the most expensive to produce of the protein amino acids. The low level of free tryptophan present in plants belies the importance of the amino acid and the pathway that produces it. In combination to the production of tryptophan needed for protein synthesis this pathway is also used to provide precursors for the synthesis of very important plant products like auxin, phytoalexins, glucosinolates, and both indole- and anthranilate-derived alkaloids. Tryptophan biosynthesis thus plays a direct role in regulating plant development, pathogen defence responses, and plant-insect interactions. Volatile indolics are also implicated in attracting pollinating animals (Radwanski and Last (1995) The Plant Cell 7: 921-934). Because this pathway provides precursors for many important other biosynthesis routes, it is difficult to manipulate the accumulation of free tryptophan without triggering other regulatory mechanisms. Increase in the free tryptophan level could be obtained by introducing a gene encoding a mutant feedback-insensitive anthranilate synthase (alpha subunit) enzyme as shown in International Patent Application WO 97/26366. However, the increase in free tryptophan levels in transgenic maize plants showed to be only 1.5 - 3 times the tryptophan levels in untransformed control plants. A high level of free tryptophan is not stable in plant cells. Tryptophan is channelled into other pathways, broken down or the synthesis is blocked due to regulatory mechanisms. Naturally occurring plant mutants with an increased free tryptophan level show only an increase of 2.5 - 3 times the wild type tryptophan. Already in 1972 it has been reported that cultured plant cell lines grown on a tryptophan analogue (5-methyltryptophan) resulted in cell lines showing an increase of 48-fold of tryptophan. However, plants could not be raised from these cell lines and resistance was lost with time when the cells were cultured without the analogue. These combined results show that due to regulatory mechanisms, instability and toxicity, it is very difficult to increase free tryptophan levels in whole plants.

All of these pathways compete for the same amino acid molecule, and it is up to the cellular environment to find a balance between the production and various uses of an available (free or unbound) amino acid molecule. Such a balance between synthesis and use, thereby regulating the concentration of an amino acid in a cell, is often provided for by a process known as feedback regulation. A well known feedback inhibition process is the feedback regulation of the synthesis of the amino acids lysine, threonine, methionine and isoleucine as for example observed in bacteria. These four are all synthesised by enzymatic biosynthesis from aspartate. Three different enzymes catalyse the initial reaction from aspartate to aspartyl phosphate, and the three are each inhibited by a different product (lysine, threonine or homoserine) from further on in the pathway. In this way it can for example happen that increased levels of one amino acid (for example methionine) inhibit synthesis of lysine or threonine. Although each amino acid is processed differently, and a whole constellation of enzyme reactions exist for their catabolism, similar feedback mechanisms rule the synthesis of other amino acids.

About half of the 20 amino acids found in proteins can be made by vertebrates; the others must be supplied in the diet. For this reason, the latter are called essential amino acids. These include the strictly essential amino acids which are lysine, leucine, isoleucine, valine, phenylalanine, methionine, threonine and tryptophan. Additionally, tyrosine and cysteine, although they are not strictly essential, must be considered as such, since they are synthesised only from essential amino acids: tyrosine from phenylalanine and cysteine from methionine. In particular, humans and other monogastric animals cannot synthesise the essential amino acids and need to obtain these from their diet. The diet of humans and livestock is largely based on plant material. However, several of these essential amino acids are often only present in low concentrations in crop plants, which mainly constitute said plant based diets. In particular, lysine, threonine, methionine or tryptophan often lack in such diets. Dietary proteins are often not nutritionally equivalent, which correlates with the amino acid composition of the different proteins. Feeding a diet that provides an inadequate amount of one of the essential amino acids leads to negative nitrogen balance, since the normal catabolism of proteins continues, but new synthesis for replacement is limited by the relative lack of the essential amino acid. This occurs even when the total dietary intake of protein is apparently adequate. The extent to which a dietary protein can be used for the synthesis of tissue proteins is limited by the content of the essential amino acid that is present in an amount relative to the requirement. This is the limiting amino acid of that protein.

Alternatively, biomass from bacterial, yeast or fungal cells may be added to a diet to increase its nutritional value by increasing amino acid content. However, even such lower organisms often produce only little of the desired amino acid(s), evolution has not designed these cells for man's purposes, and consequently even diets supplemented with the above described biomass cultures are often lacking in at least one of the essential amino acids. Therefore, synthetic amino acids are often added as supplement to for example grain- or other plants-based diets, in order to increase the nutritional value of the feed.

Various attempts have been made in the past to increase the level of essential amino acids in plants by classical breeding techniques or by mutant selection. The general idea being that the genetically improved plant would, if containing more of a desired amino acid and thus having higher nutritional value, be a promising candidate to be incorporated in the diets of humans or livestock. However, these attempts have met with little success, most likely because, albeit being very desirable for mankind to obtain plant types enriched for one or more (essential) amino acids, plants have little use, and more likely experience detrimental effects, from those very same increased amino acid levels, which severely decreases the chances of obtaining such plants by classical breeding, mutating or selection techniques.

More recently, however, modern biotechnological or recombinant techniques have allowed to equip plants with means for overproducing a desired amino acid, by providing one or more additional essential amino acid biosynthesis enzymatic pathways, purportedly leading to increased levels of free essential amino acids in the cell. These transformed or transgenic plants seem thus in first instance promising candidates to be incorporated in the diets of humans or livestock.

However, such transgenic plants with increased production of free amino acid suffer in general from various drawbacks, which, in essence, are related to that same increased amino acid production capacity. For example, it turns out that increasing the production levels of one, say lysine, by providing the plant with the necessary extra enzymatic biosynthesis pathway for that specific amino acid, effected (for example by the above described feedback regulation) decreased levels of another, say threonine or methionine. Also, increased levels of various free amino acids in a plant cell, such as lysine or tryptophan, are soon considered reaching toxic levels, which ultimately results in decreased production capacity of said plant. Furthermore, as a consequence of raising levels of concentration of an available amino acid molecule, various cellular pathways act readily by using the increased substrate levels for their own purposes, for example the extra amino acid is often simply oxidised to generate metabolic energy and thus gets lost. Regulation of amino acid biosynthesis is mainly based on feedback inhibition of the key enzymes from the pathway by the amino acid end products. Problems encountered by increasing the essential amino acid content in plants are due to the feedback inhibition mechanism. Furthermore, high levels of free amino acids in plant cells can be toxic to the plant (Shaul, O. and Galili, G. (1992a) Plant J. 2: 203-209; Frankard, V. et al. (1992) Plant Physiol. 99: 1285-1293). Another problem that can be encountered is that the increase of one amino acid goes at the expense of the accumulation of another amino acid. For example, by molecular genetic modification it was attempted to increase both lysine and threonine simultaneously in the same transgenic plant. Increase of free lysine proved to operate at the expense of the accumulation of free threonine (Shaul, O and Galili, G (1993), Plant Mol. Biol. 23:759-768; Falco, S.C. et al. (1995), Bio/Technology 13: 577-582).

In essence, biosynthesis of the 10 essential amino acids can be categorised into three groups: (1) amino acids synthesised from aspartate, comprising lysine, methionine (and cysteine) and threonine (the aspartate-family amino acids), (2) the branched chain amino acids, comprising leucine, isoleucine and valine, and (3) the aromatic amino acids, comprising phenylalanine, tyrosine and tryptophan. Biosynthesis pathways for all amino acids can be found in any biochemical textbook (e.g. Stryer, L.(1975), W.H. Freeman and Company, San Francisco; Bender, D.A. (1985), John Wiley & Sons, New York). Biosynthesis pathways for tryptophan and the aspartate-family amino acids are given below as an example.

### Biosynthesis of tryptophan

Tryptophan is one of the three aromatic amino acids. Phenylalanine and tryptophan are essential amino acids, while tyrosine can be synthesised by hydroxylation of phenylalanine. The initial stages of synthesis are common to all three aromatic amino acids. After the forming of chorismic acid the pathway of tryptophan formation diverges from that for phenylalanine and tyrosine. Anthranilate synthase (AS) catalyses the first reaction branching from the aromatic amino acid pathway to the biosynthesis of tryptophan. This reaction is the conversion of chorismate to anthranilate in a glutamine dependent reaction. The next reaction in the synthesis of tryptophan is the transfer of the phosphoribosyl moiety of phosphoribosyl pyrophosphate to anthranilate. The indole ring is formed in two steps involving an isomerisation converting the ribose group to a ribulose followed by a cyclisation reaction to yield indole glycerol phosphate. The final reaction in the pathway is catalysed by a single enzyme that may contain either one or two subunits. The reaction accomplishes the cleavage of indole glyceraldehyde-3-phosphate and condensation of the indole group with serine (Umbarger (1978) Ann. Rev. Biochem. 47: 555). Metabolite flow in the tryptophan pathway in higher plants is among others regulated through feedback inhibition of anthranilate synthase by tryptophan.

### Biosynthesis of aspartate-family amino acids

The essential amino acids lysine, threonine and methionine are synthesised from aspartate by a complex pathway which is similar for bacteria and higher plants. The aspartate family pathway has been characterised in detail in *Escherichia coli* by isolation of enzymes involved in the pathway, which were later also purified from higher plants (Bryan, J.K. (1980), The Biochemistry of Plants (ed. by B.J. Miflin) Vol. 5: 403-452, Academic Press, N.Y.; Umbarger, H.E. (1978) Ann.Rev.Biochem. 47: 533-606).

The rate of synthesis of the aspartate-family amino acids is regulated primarily by a complex process of feedback inhibition of the activity of some key enzymes in the pathway by the relevant amino acid end product. The first enzymatic activity in the pathway that is common to all of the aspartate-family amino acids, aspartate kinase (AK) activity, is feedback inhibited by both lysine and threonine. In addition, lysine also inhibits the activity of the enzyme dihydrodipicolinate synthase (DHPS), the first enzyme of the pathway after the branch point that leads to the synthesis of lysine. Threonine inhibits the activity of homoserine dehydrogenase (HSD), the first enzyme involved in the biosynthesis of threonine (Matthews, B.F. et al. (1989) Plant Physiol. 91: 1569-1574).

The enzyme aspartate kinase (AK) catalyses the phosphorylation of aspartate to form 3-aspartyl phosphate, with the accompanying hydrolysis of ATP. Both in *E. coli* and in plants several different AK isoenzymes have been identified which are differentially inhibited either by lysine or by threonine. AK-III, the product of the *E. coli* lysC locus is shown to consist of two identical subunits as a homo-dimer (Casan, M. et al. (1986), J.Biol.Chem. 261: 1052-1057; Richaud, C. et al.( 1973) Eur.J.Biochem. 40: 619-629). The *E. coli* LysC gene has been cloned and sequenced (Cassan, M. et al. (1986), see above).

The product of AK activity, 3-aspartyl phosphate, is in the next enzymatic step converted to 3-aspartic semialdehyde (3-ASA), which serves as a common substrate for the synthesis of both lysine and threonine. The enzyme dihydrodipicolinate synthase (DHPS) catalyses the first reaction that is unique to lysine biosynthesis, the condensation of 3-aspartate semialdehyde with pyruvate to form 2,3-dihydrodipicolinate. In *E. coli* this enzyme is encoded by the dapA locus and appears to consist of four identical subunits as a homotetramer (Shedlarski, J.G. and Gilvarg, C. (1970) J.Biol.Chem. 245: 1362-1373). The *E. coli* dapA gene has been cloned and sequenced (Richaud, F. et al. (1986) J.Bacteriol. 166: 297-300). In plants DHPS enzyme also appears to be a single enzyme comparable to the E.coli enzyme. Among the major regulatory enzymes of the aspartate family pathway in plants, DHPS is the most sensitive to feedback inhibition by its endproduct (I₅₀ of DHPS for lysine ranges between 10 and 50 µM). DHPS is about 10-fold more sensitive to lysine inhibition than are plant lysine-sensitive AKs (I₅₀ between 100 and 700 µM) and about 100-fold more sensitive to lysine inhibition than *E. coli* DHPS (I₅₀ is about 1mM) (Yugari, Y. and Gilvarg, C. (1962) Biochem.Biophys.Acta 62: 612-614; Galili, G. (1995) The Plant Cell 7: 899-906).

Homoserine dehydrogenase (HSD) catalyses the first reaction that is specific for the synthesis of threonine, methionine and isoleucine. Higher plants generally possess at least two forms of HSD: a threonine-sensitive form and a an insensitive form (Bryan, J.K. (1980) The Biochemistry of plants (ed. by B.J. Miflin) 5: 403-452, Academic Press, N.Y.; Lea, P.J. et al. (1985) Chemistry and Biochemistry of the Amino Acids (ed. by G.C. Barrett): 197-226, London: Chapman and Hall).

Several lines of evidence have indicated that in plants AK is the rate-limiting enzyme for threonine synthesis, while DHPS is the major rate-limiting enzyme for lysine synthesis. Mutants of several plant species possessing feedback-insensitive AK isozyme were found to overproduce free threonine, but exhibited only a slight increase in the level of lysine (Bright,S.W.J. et al. (1982) Nature 299: 278-279; Cattoir-Reynaerts A. et al. (1983) Biochem.Physiol.Pflanzen 178: 81-90; Dotson, S.B. et al. (1990) Planta 182: 546-552; Frankard, V. et al. (1992) Plant Physiol. 99: 1285-1293). On the other hand, a feedback-insensitive DHPS mutant tobacco plant overproduced lysine (Negrutiu, I. et al. (1984) Theor.Appl.Genet. 6: 11-20). Similar results have been reported with transgenic plants expressing feedback-insensitive DHPS or AK from *E. coli* (Glassman, K.F. (1992) Biosynthesis and Mol. Regul. of Amino Acids in Plants (ed. by B.K. Singh et al.): 217-228; Perl, A. et al. (1992) Plant Mol.Biol. 19: 815-823; Shaul, O. and Galili, G. (1992a) Plant J. 2: 203-209; Shaul, O. and Galili, G. (1992b) Plant Physiol. 100: 1157-1163). Transgenic plants that expressed the *E*. *coli* DHPS overproduced lysine, while those that expressed the *E. coli* AK overproduced threonine and exhibited only a slight increase in the level of lysine. Studies of transgenic plants have also demonstrated that AK and DHPS are not only regulated by feedback inhibition, but also the levels of these enzymes limit the rate of production of threonine and lysine. In transgenic plants a significant positive correlation was detected between the levels of the bacterial AK and DHPS enzymes and the levels of free threonine and lysine, respectively (Shaul, O. and Galili, G. (1992 a and b), see above).

However, transgenic plants expressing both a feedback-insensitive AK and a feedback-insensitive DHPS (Shaul, O. and Galili, G. (1993) Plant Mol. Biol. 23: 759-768) contain free lysine levels that far exceed those in plants expressing only the introduced insensitive AK or DHPS. This lysine increase is also accompanied by a significant reduction in threonine accumulation as compared with transgenic plants expressing the insensitive AK only. This means that when DHPS becomes deregulated the branch leading to lysine synthesis competes strongly with the other branch of the pathway and a considerable amount of 3-aspartic semialdehyde is thus converted into lysine at the expense of threonine. The same results were obtained by crossing a lysine overproducing mutant with a threonine overproducing mutant characterised by an altered regulation of respectively DHPS and AK (Frankard, V. et al. (1992) Plant Physiol. 99: 1285-1293).

European Patent Application No. EP 485.970 discloses a method of increasing the level of both free threonine en lysine by introducing into plant cells a first chimeric gene comprising a DNA sequence coding for an enzyme having AK activity and a second chimeric gene comprising a DNA sequence coding for an enzyme having DHPS activity. Both chimeric genes further comprise DNA sequences enabling expression of the enzymes in the plant cells and subsequent targeting of the enzymes to the chloroplast.

European Patent Application No. EP 93908395 describes two isolated DNA fragments comprising a fragment encoding AK insensitive to inhibition by lysine and a second fragment encoding DHPS which is at least 20-fold less sensitive to inhibition by lysine than plant DHPS. It is claimed that the lysine-insensitive AK causes a higher than normal threonine production and that the DHPS causes a higher than normal lysine production in transformed plants.

However, while it is shown that transgenic plants expressing feedback-insensitive DHPS from *E. coli* overproduce lysine and that transgenic plants expressing the E. coli AK overproduce threonine (Glassman, K.F. (1992) Biosynthesis and Mol. Regul. of Amino Acids in Plants (ed. by B.K. Singh et al.): 217-228; Perl, A. et al. (1992) Plant Mol.Biol. 19: 815-823; Shaul, O. and Galili, G. (1992a) Plant J. 2: 203-209; Shaul, O. and Galili, G. (1992b) Plant Physiol. 100: 1157-1163), combining these two genes in transgenic plants never resulted in a comparable increase of both threonine and lysine. Shaul and Galili (1993) Plant Mol. Biol. 23: 759-768) obtained transgenic plants expressing both feedback-insensitive AK and feedback-insensitive DHPS by crossing transgenic plants that expressed each of these enzymes individually. These plants were shown to contain free lysine levels that far exceeded those in plants expressing only the insensitive DHPS. The lysine increase however was accompanied by a significant reduction in threonine accumulation as compared with plants expressing the insensitive AK only.

The same results were found when feedback-insensitive bacterial DHPS and AK enzymes encoded by the *Corynebacterium* dapA gene and a mutant *E. coli* lysC gene, respectively, were expressed together in transgenic canola and soybean seeds. Several hundred-fold increases in free lysine in transgenic seed was observed, whereas the accumulation of excess threonine that was seen in transgenic seed expressing feedback-insensitive AK alone was prevented by the co-expression of DHPS (Falco,S.C. et al. (1995), Bio/Technology 13: 577-582).

These findings suggest that the ratio between lysine and threonine synthesis in plants is regulated by at least two factors. First, the availability of 3-ASA which is the common substrate for the two key-enzymes specific for threonine and lysine synthesis (respectively homoserine dehydrogenase and DHPS). Second, the competition between these two key-enzymes for 3-ASA as a common substrate. The level of 3-ASA seems to be determined by the activity of AK. When feedback-insensitive AK is overexpressed in transgenic plants, the higher 3-ASA concentration can be channelled into the threonine synthesis branch. However, when DHPS becomes feedback-insensitive the branch leading to lysine synthesis competes strongly with the other branch of the pathway and a considerable amount of 3-ASA is thus converted into lysine at the expense of threonine synthesis. In order to increase the amount of both free lysine and threonine in transgenic plants to desirable levels, the timing and the level of expression of each introduced feedback-insensitive gene should be highly regulated.

Furthermore, abnormal phenotypes were observed in transgenic plants in which the free lysine concentration is increased in the whole plant to more than 10-fold the concentration found in untransformed plants (Glassman, K.F. (1992) Biosynthesis and Mol. Regul. of Amino Acids in Plants (ed. by B.K. Singh et al.): 217-228; Shaul, O. and Galili, G. (1992a) Plant J. 2: 203-209; Frankard, V. et al. (1992) Plant Physiol. 99: 1285-1293).

In European Patent Application No. EP 485970 the expression of both feedback-insensitive AK and DHPS genes was driven by the same constitutive CaMV35S promoter. Also in the experiments of Shaul and Galili ((1993) Plant Mol.Biol. 23: 759-768) and of Falco et al. ((1995), Bio/Technology 13: 577-582) in which both feedback-insensitive AK and DHPS were co-expressed in transformed plants, the same promoter was used to drive expression of the two genes.

In the light of above, a need exists to better regulate free amino acids levels in a plant cell, or its contents, that may be added to a diet to increase its nutritional value. Most notably, the availability of the desired amino acid in such a cell need be better guaranteed, whereby it should preferable be kept in mind that the availability of one does not hamper the availability of another amino acid, of which, by feedback regulation, less than desired may be produced.

The invention provides a method for regulating the supply, herein also called concentration, flux or biosynthesis of a desired amino acid in a cell comprising providing said cell with a nucleic acid encoding a proteinaceous substance comprising at least said amino acid, such a proteinaceous substance herein also called sink protein whereby sink stands for the capacity of said proteinaceous substance to function as sink or reservoir for the desired amino acid, thereby creating a demand in said cell for said amino acid thereby regulating or reducing the amount or concentration of available of free amino acid in said cell by binding it through transcription and translation processes to the sink protein encoded by the additional nucleic acid.
Proteinaceous substance herein is used in a general sense, it comprises any small or large (poly)peptide or protein, e.g. any multitude of, but at least two, amino acids linked by amide linkage or peptide bond, which substance may or may not be further modified by commonly known additions or modifications such as glycosylation, acetylation, methylation, phosphorylation, covalent linkage, hydrolysis, digestion or any other protein or peptide modifications which said cell is capable of. Also it is provided by the invention to provide said proteinaceous substance with commonly known signal sites, such as protein or peptide targeting or translocation signals by which said substance, after biosynthesis, is routed from its site of origination to other sites or compartments in said cell where it is located, aggregated or stored. Other sites that are advantageously incorporated in said proteinaceous substance or sink protein as provided by the invention comprise protein cleavage signal sites, whereby said proteinaceous substance, after biosynthesis, are cleaved in smaller parts, such as peptides which find easy storage in said cell. Signal sites, such as targeting signals or protein cleavage signals are well known in the art, and are easily provided for by incorporating the appropriate nucleic acid codons (encoding the appropriate amino acids in the desired order) in the nucleic acid encoding the sink protein. The invention for example provides a method for regulating the concentration of an amino acid in a cell comprising providing said cell with a nucleic acid encoding a proteinaceous substance comprising at least said amino acid, wherein said cell, or (micro)-organism comprising said cell, is further subjected to classical breeding, mutating or selection techniques, thereby providing a method allowing to obtain cells or (micro)-organisms such as bacterial, yeast, fungal or plant types enriched for one or more (essential) amino acids which do not experience detrimental effects from said increased levels. These cells or organisms have been provided with a sink protein as provided by the invention which functions as sink or reservoir for the desired amino acid, thereby regulating biosynthesis or reducing the amount or concentration of available of free amino acid in said cell or cells of said organism by binding it through transcription and translation processes to the sink protein encoded by the additional nucleic acid with which it is provided. A sink protein as provided or used according to the invention preferably contains about at least 7,5% lysine, or 2.5% methionine, or 2.5% cysteine, or 1.5% tryptophan, or 6% threonine, or 6.5% valine, or 12.5% leucine, or 5.5% isoleucine, or 6.5% phenylalanine, or 5.5% tyrosine.

In a preferred embodiment of the invention, a method according to the invention is provided further comprising providing said cell with a nucleic acid allowing enhanced translation of said proteinaceous substance or sink protein. The problems that can be encountered trying to increase free essential amino acid levels in plants, as described above for tryptophan, threonine, lysine and methionine, also hold for the other essential amino acids. In the present invention it is provided to for example both increase the level of free essential amino acid and subsequently trap that free amino acid in a sink protein by which it is stabilised and removed from the feedback-mechanism. An enhanced production of this sink protein increases both the protein amount and the protein composition of the transformed plants.

Preferably, the invention provides a combination of coordinated expression in a cell of nucleic acid encoding at least one feedback insensitive amino acid biosynthesis enzyme in combination with a nucleic acid encoding a sink protein, wherein the desired amino acids are incorporated and removed from the, as yet unbound, pool of free amino acids, thereby creating an enhanced flux of desired amino acid from a free to an incorporated form. In addition, enhanced production of sink protein, thereby even greater enhancing said flux, can be achieved by providing enhanced translation of said sink protein.

Sink protein candidates can for example be selected from among known storage proteins. Several publications describe the amino acid composition of plant storage proteins, and their possible use to enhance the essential amino acids composition of food and feed crops. The storage proteins of cereal crops like wheat, barley and maize of the so-called prolamin type vary in their content of sulphur-containing amino acids (methionine and cysteine). Some are relatively high in S-rich amino acids. However, most of them are severely deficient in lysine and tryptophan (Shewry, P. R., (1998) Transgenic Plant Research, p.135-149). The storage proteins in legumes and other dicotyledons are mainly of the globulin family or the albumin family. Globulins are generally very poor in the sulphur-containing amino acids, but sometimes do contain a relatively high ratio of lysine. Vicilin (of *Vicia faba)* has a lysine content of 7.2%, threonine content of 3% and a methionine content of only 0.2%. The 2S albumin family of storage proteins in general have a high content of S-rich amino acids. Brazil nut 2S albumin contains ca. 26% sulfur amino acids (Ampe 1986), and sunflower 2S albumin (Sfa8) contains 24% sulfur amino acids (Kortt 1991).

Potato tubers in general have a limiting composition of methionine and cysteine (Van Gelder, W.M.J. and Vonk, C.R. (1980) Potato Res. V23 427-434). The storage protein of potato, patatin has a reasonably high content of lysine (7%) and threonine (9%), and a low content of methionine (2.6%) and tryptophan 0.5%).

Attempts to improve the nutritional quality of feed crops like legumes and canola by transforming with a gene construct encoding a storage protein have been reported. The results were in most cases disappointing, since the level to which the introduced gene product accumulated in the transgenic plant was very limiting. In most cases the level of accumulation was around or below 1% of the total protein content (Saalbach 1990, 1995 Molecular breeding V1 245-258, Utsumi (1994) Plant Science V102, 181-188).

Some encouraging results have been obtained by transforming plants with gene constructs encoding a 2S albumin, from either Brazil nut or sunflower (Saalbach 1994; MGG V242, 226-236 and Saalbach 1995b J. of Plant Phys. V145, 674-681). The level of Brazil nut albumin reached up to 6% of total protein, making use of a tissue specific promoter. However, in later studies it appeared that the Brazil nut albumin is strongly allergenic. The sunflower albumin appears to have better characteristics in this respect (Tabe, L.M. et.al. (1993) Genetica V90 p181-200). Another interesting storage protein with a very high content (40%) of sulphur amino acids is the homologue to the 10kD zein from maize (Chui and Falco (1995) Plant Phys. V107, 291). This protein contains an internal repeat domain containing more than 50% methionine.

Other storage proteins that have a high content of lysine residues are the protease inhibitors C1 and C2 from barley (9.5% and 11.5% lysine respectively, Hejgaard and Boisen (1980)). Transgenic plants transformed with these genes have not yet been described, but they provide a good sink protein for increased lysine levels when incorporated in a cell as provided by the invention.

Yet other approaches have aimed at improving the amino acid composition of storage proteins, in order to enhance the level of essential amino acids. Wallace et al. (1988. Science V240, 662-664) have modified α-zein by incorporating small stretches of lysine and tryptophan or by making a fusion to a lysine rich protein. These modified α-zein proteins retained the ability to accumulate as protein bodies, even in a heterologous system as Xenopus oocytes. Saalbach et. al.(1995) (Molecular Breeding V1 245-258) reported on the use of modified gene constructs encoding vicilin and legumin with additional stretches of methionine. In the transformed plants the modified legumin was quickly degraded, whereas the modified vicilin could accumulate, albeit to lower levels than the unmodified protein. Also in other publications, modified storage proteins often accumulate to much lesser amounts than the wild type protein (Hoffman, L.M. et. al. (1988) Plant Mol. Biol. V11, 717-730).

To enhance sink protein production, the invention provides providing said cell with a nucleic acid allowing enhanced translation of said sink protein. In a preferred embodiment, the invention provides a method wherein an enhancer sequence such as an omega-sequence, or likewise, a translational enhancer is at least functionally linked (in *cis* or *trans)* to the coding sequence of this storage protein, said sequence enabling high expression of the sink protein gene, either in the whole cell or organism or in specific parts or tissues of the organism. Such nucleic acid is for example DNA coding for the translational protein factor, like hsp101, which recognises the enhancer sequence in the mRNA from a sink protein, promoting its translation and also DNA sequences enabling expression of a translational protein factor, like hsp101 in cells in which the sink protein is expressed.

The invention furthermore provides a method according to the invention further comprising providing said cell with a nucleic acid encoding at least one functional enzyme related to said amino acid's biosynthesis pathway allowing said cell to increasingly synthesise said amino acid, and thereby further regulates supply. In a preferred embodiment, said amino acid is an essential amino acid, such as tryptophan, methionine, threonine or lysine. For example, in one embodiment of the invention a method is provided wherein one or more gene constructs or nucleic acid molecules capable of expression in a plant cell with subsequent increased production of both free threonine and lysine is given, followed with an enhanced incorporation of these essential amino acids into a sink protein fraction of the plant cell. Free amino acid level is increased by introducing at least one gene encoding (a feedback insensitive) enzyme involved in biosynthesis of said amino acid. The over-produced free essential amino acids are trapped by incorporation in a sink protein, rich in said essential amino acid, that is expressed at the same time in the plant. Preferably, the translation of this sink protein is specifically enhanced by simultaneous expression of a protein factor which recognises a sequence domain in the messenger RNA that is produced from the sink protein gene, and as such enhances the efficiency of protein synthesis of this specific protein, thereby regulating biosynthesis or concentration of said amino acid.

The invention furthermore provides a transgenic cell having been provided with a nucleic acid encoding a proteinaceous substance, a sink protein, comprising at least a desired amino acid allowing regulating the concentration of said amino acid in said cell. In a preferred embodiment, said cell has further been provided with a nucleic acid allowing enhanced translation of said sink protein. In a preferred embodiment, said cell accumulates said sink protein up to more than 2%, preferably 4%, or even more than 5% to more than 7% of the total protein content of said cell. Such levels of expression are also reached by accumulation of sink protein in aggregates or more-or-less regular crystal-like structures. Preferably, said cell has additionally been provided with a nucleic acid encoding at least one functional enzyme related to said amino acid's biosynthesis pathway allowing said cell to increasingly synthesise said amino acid, preferably wherein said amino acid is an essential amino acid and thereby further regulates supply.

The invention furthermore provides an organism derived from a cell according to the invention. Such an organism is for example a micro-organism, such as a yeast, fungus or bacteria, suitable for incorporation as a whole or at least in part in animal foodstuff, thereby increasing the nutritional value of said foodstuff. Especially when an amino acid is overproduced by said organism, said nutritional value is greatly increased, especially for monogastric animals such as humans, pigs or fish, or other animals, such as poultry, that do not or only little rely on bacterial conversion of foodstuff as source of essential amino acids. In a preferred embodiment of the invention an organism is provided which is a plant, preferably a crop plant such as potato, sugar beet, carrot, cassave, canola, alfalfa, legumes, or gramineae species like rice, maize, wheat, sorghum, barley or grass. Harvesting a crop plant is a skill known in the art, harvesting a plant as provided by the invention allows harvesting foodstuff material with increased nutritional value.

In yet another embodiment, the invention provides foodstuff for an animal comprising at least part of a cell or organism according to the invention, said foodstuff is particularly useful when said animal is monogastric. Feeding such an animal with a foodstuff as provided by the invention allows providing a positive effect on the nitrogen balance of said animal, it receives better balanced food allowing optimal use of the available protein matter, thereby reducing its nitrogen spoilage. Furthermore, the invention also provides a food animal product derived from an animal fed a foodstuff according to such an animal. From such an animal in general better balanced food animal products are derived. As a result of less protein spoilage a better product is obtained, e.g. less calories or energy are wasted on fat deposition. The invention furthermore provides use of at least part of a cell or organism according to the invention for increasing nutritional value of a foodstuff. The invention is further explained in the detailed description without limiting the invention.

### Detailed description

A method is provided to increase a desired amino acid level in micro-organisms or organisms such as plants. (Over)produced free (essential) amino acids are trapped by incorporation in a sink protein, rich in said amino acid, that is expressed at the same time in the plant. Optionally, the translation of this exogenous sink protein is specifically enhanced by simultaneous expression of a protein factor which recognises a sequence domain in the messenger RNA that is produced from the sink protein gene, and as such enhances the efficiency of protein synthesis of this specific protein. Free amino acid level may be increased by introducing at least one gene encoding (a feedback insensitive) enzyme involved in biosynthesis of said amino acid. In the case of the aspartate-family the problem of decrease in threonine accumulation as a result of lysine overproduction, may for example be circumvented by differentially expressing AK and DHPS. A very strong constitutive promoter is used to drive feedback-insensitive AK expression throughout the whole plant during all stages of organ development, resulting in a higher 3-ASA concentration which can be channelled into the threonine synthesis branch and will lead to threonine overproduction. Subsequently, the feedback-insensitive DHPS enzyme is only expressed at a lower level in specific plant organs. Because the expression of DHPS is at a later stage and at a lower level, the branch leading to lysine synthesis competes only weakly and during a well defined period of development with the other branch of the pathway and both threonine and lysine will be overproduced in the targeted plant organs. Because lysine is only overproduced in specific plant organs like tubers, the rest of the plant will develop normally without showing aberrant phenotypes.

### Ad 1) Increasing essential amino acids biosynthesis in plants

The free levels of other essential amino acids, separately or in combination, can be increased in essentially the same way as described above. For instance, to increase the level of free lysine a gene encoding a bacterial or plant feedback-insensitive DHPS can be used. To elevate free cysteine levels a bacerial or plant gene encoding serine acetyltransferase (SAT) could be expressed in plants. A combination of cysteine with methionine increase can be obtained by combining expression of SAT encoding gene together with a plant or bacterial gene encoding the enzyme cystathionine gamma synthase (CgS). Methionine levels can also be elevated by introducing genes encoding two enzymes, for example obtainable from yeast, homoserine acetyltransferase (HAT) or acetylhomoserine sulfhydrolase (AHS), catalysing a methionine pathway which is in general not present in plants. Also, to increase the level of free tryptophan a gene encoding a mutant (bacterial or plant) feedback-insensitive enzyme with anthranilate synthase activity is introduced and expressed in the plant. This gene is for example a TrpE mutant from *Salmonella typhimurium* or a mutant of the *Arabidopsis thaliana* anthranilate synthase alpha subunit gene for example in combination with the *Arabidopsis thaliana* anthranilate synthase beta subunit gene (J. Li and R.L. Last, Plant Physiol. 110:51-59 (1996) M.G. Caligiuri and R. Bauerle, J. of Biol. Chem. 266: 8328-8335 (1991) ). For another essential amino acid a corresponding gene encoding a (mutant), preferably feedback-insensitive, key-enzyme of the corresponding pathway is introduced.

### Ad 2) Proteins enriched in essential amino acids

The amino acid composition of the total protein fraction is determined by the amino acid content of the individual proteins combined with the relative abundance of each protein within the total population. In the total protein fraction of plants the abundance of essential amino acids like methionine, lysine, tryptophan, cysteine or threonine are relatively low. In order to trap the overproduced lysine and threonine (see ad 1) and remove them from the feedback regulation mechanism, and also to increase the relative abundance of essential amino acids within the total protein fraction, the invention provides introducing a gene encoding for a protein that is exceptionally enriched in the desired essential amino acids. This protein is expressed at high levels to represent a major fraction of the total protein pool, in order to have effect on the total amino acid composition. The high production of the essential amino acids protein functions as a sink for the incorporation of these desired essential amino acids and removes them from the feedback mechanism, and as such increases the level of biosynthesis of the essential amino acids.

### Selection of proteins

There are several selection criteria to which the sink protein preferably conforms.

The protein preferably contains a high content of essential amino acids (preferably methionine, cysteine, lysine, threonine, or tryptophane) in such a ratio as to correct for the lack of these essential amino acids in the amino acids composition of the total protein fraction of the tissue. As food or feed organisms, or tissues, differ in limiting essential amino acids, the optimal amino acid content for a sink protein varies according to organism. A sink protein preferably is a protein specifically enriched in those amino acids for which a deficite occurs in the specific crop or organism. By producing the sink protein to at least 2%, preferably to at least 4%, 5%, or even at least 7% of the total protein content of the tissue which is being used as food or feed, we compensate for the essential limiting amino acid. For example, for potato a sink protein preferably contains at least 5%, more preferably at least 10% lysine, at least 2.5% methionine, at least 2.5% cysteine, or at least 1.5% tryptophan. For maize, 2.5% to 5% lysine or 1% tryptophane is considered efficient.

The protein is stable in the plant, accumulates to high levels and has no drastic detrimental effects on the growth and physiology of the crop plant. The protein is well digestible by the livestock and/or human digestive tract. Optionally it has some health promoting quality.

### Protein stability and aggregation

The level of protein accumulation in a plant is determined by the rate of synthesis in relation to the rate of degradation of this protein. The rate of degradation is determined by its sensitivity to attack of proteases that are present in the producing tissue. This protease sensitivity is influenced by the availability of susceptible sequence domains on the surface of the protein, in combination with the structural rigidity of the protein. In order to select for a protein that will have a high chance to accumulate in the plant, the protein preferably has a rigid tertiary structure, with minimal exposed sequence domains. Certain proteins have a native tendency to aggregate into more or less regular or organised macromolecular structures, such as protein bodies or protein crystals. Naturally, storage proteins that accumulate in plant tissue, where they have a storage function, are naturally adapted to remain stable in these plant tissues. Therefore seed storage proteins are distinct candidates to accumulate essential amino acids. However, few plant storage proteins will always have a desired composition relating to the desired essential amino acids. Furthermore, in general the amount of essential amino acids is often too low. The invention herewith provides using (semi-)synthetic storage or sink protein that is encoded by a nucleic acid enriched with the necessary codons encoding said desired amino acids. In addition to this, in nature several proteins exist that form (semi)-crystalline structures in their natural tissue. Examples are some peroxisomal proteins like alcohol oxidase or urate oxidase, or crystallins (eye lens proteins). Also viral structural proteins are able to form regular crystal like structures, that function to protect the interior of the virus to many types of biotic and abiotic attack. The invention provides (semi-)synthetic proteins that, based on a regular molecular structure, are able to form stable, more or less regular crystal-like structures.

### Ad 3) Specific enhanced translation of the introduced sink protein

It has been demonstrated that the presence of viral RNA leader sequences present as fusion linked to heterologous messenger RNA's can significantly enhance the translation of this chimeric messenger RNA into proteins, when expressed in tobacco plants. (Gallie, PMB '96, Gallie D.R. (1996), Plant Mol.Biol. 32:145-158 and NAR '87 V15, pp. 3257-73) The mechanism of this translational enhancement was originally explained by the lack of secondary structure in the messenger leader, and the possible complementarity to ribosomal RNA. Recently, however, it has been shown for the Omega enhancer sequence, which is present at the 5' untranslated region of Tobacco Mosaic Virus (TMV), that a protein factor binds to this sequence (Tanguay & Gallie '96, J.B.C.V271, p14316-22). This protein factor has been identified as hsp101. It was shown to bind to the TMV 5' and also the 3' UTR. In a heterologous yeast expression system the co-expression of tobacco hsp101 was shown to enhance the translation of a chimeric Q-Luc reporter mRNA by a factor 7 to 10 fold (D.R Wells et.al. Genes&Devel. '98, V12, p3236-51). The hsp101 of wheat was shown to have a similar stimulatory effect on the translation of omega-enhancer containing messenger but to a much lower extent.
We describe the co-ordinate expression of hsp101, from a cDNA isolated from tobacco, brought under control of a plant tissue specific promoter. The combination of this expression cassette with an expression cassette encoding for a sink protein, operationally linked to the Omega enhancer sequence, results in an increased expression of the sink protein due to the selective translational stimulation by the hsp101 protein. This approach will also be useful for the enhancement of any other type of heterologous protein, introduced by gene transformation into the plant.

In one embodiment, the present invention is directed to a combination of gene constructs capable of expression in specific plant cells with subsequent increased production of both free threonine and lysine to the same level, and with an enhanced incorporation of these essential amino acids into the protein fraction of the plant cells. Such a combination of gene constructs in a plant cell as provided by the invention comprises: (a) a DNA sequence coding for an enzyme having AK activity, (b) DNA sequences enabling high expression of the AK enzyme in all plant cells or plant organs at an early stage during organ development and subsequent production of high levels of threonine, (c) a DNA sequence coding for an enzyme having DHPS activity, and (d) DNA sequences enabling expression of this DHPS enzyme in specific plant cells or plant organ and at a lower level and at a later stage during development compared to the AK expression and subsequent production of high levels of lysine, (e) a DNA sequence coding for a storage protein rich in essential amino acids or a sink protein forming (semi)-crystalline structures, (f) an omega-sequence, or likewise, translational enhancer functionally linked to the coding sequence of this storage protein, (g) DNA sequence enabling high expression of the storage protein gene, either in the whole plant or in specific tissues of the plant (h) DNA coding for the translational protein factor, like hsp101, which recognises the enhancer sequence in the mRNA from the storage protein, promoting its translation and (i) DNA sequences enabling expression of the translational protein factor, like hsp101 in all plant cells in which the storage protein is expressed.

The invention also relates to expression vectors comprising the different combinations of the gene constructs of the invention.

In another embodiment, the invention provides transgenic plant cells containing four chimeric genes, for example encoding AK, DHPS, sink protein and translational protein factor, according to the invention or transformed by expression vectors according to the invention, as well as transgenic plants producing in specific organs high levels of both free threonine and lysine and bound into the protein fraction regenerated from said plant cells, and tissues derived from said transgenic plants.

One object of the invention is to generate plants that overproduce both free threonine and lysine to the same extent and additionally trap these essential amino acids in a highly expressed sink protein containing a high amount of these essential amino acids, by transformation of plant cells with the said chimeric quarternary gene construct.

The DNA sequences coding for an enzyme having amino acid biosynthesis activity are derivable from any suitable source, e.g. from different plant cells or from bacteria, or can even be a synthetic gene. The preferable exogenous sources are bacteria, e.g. *E. coli,* for AK is preferred a DNA sequence encoding AK activity such as the DNA sequence of *E. coli* mutant LysC gene coding for the isoenzyme AK-III, which is less sensitive to lysine than the plant enzyme (Casan, M. et al. (1986), J.Biol.Chem. 261: 1052-1057).

A preferred DNA sequence coding for a DHPS enzyme used according to the invention is the dapA gene from E. *coli* (Richaud, F. et al. (1986) J.Bacteriol. 166: 297-300) which has a DNA sequence coding for a DHPS enzyme which is less sensitive to lysine inhibition than the plant DHPS enzyme.

The DNA sequences enabling expression of the enzymes AK and DHPS in plant cells include plant promoters of different kinds, which especially differ from each other in strength and in timing of activity. The promoters may origin from both mono- and dicotelydoneous plants, preferably tissue specific but also non-tissue specific promoters.

A preferred promoter to drive expression of the AK enzyme according to the invention is the enhanced cauliflower mosaic virus (CaMV) 35S promoter (Benfey, P.N. et al. (1990) EMBO J. 9: 1685-1696; Pen, J. et al. (1992) Bio/Techn. 10: 292-296). In this promoter the enhancer sequence is duplicated which turns it into a strong promoter which drives expression constitutively throughout the whole plant in all stages of development. Any other promoter which drives constitutive expression at a high level can be used. But also the same tissue-specific promoter that regulates for example DHPS expression can be used to drive feedback-insensitive for example AK expression when fused to an enhancer.

Another preferred promoter to drive expression of an in amino acid biosynthesis involved enzyme according to the invention is the tuber-specific class I patatin promoter from *Solanum tuberosum* (Mignery, C.A. et al. (1988) Gene 62: 27-44; Wenzler, H.C. et al. (1989) Plant Mol.Biol. 12: 41-50). Other tuber-specific promoters can be used to drive enzyme expression like the granule bound starch synthetase (GBSS) promoter (Visser R. (1991) PlantMol.Biol 17: 691-699 (1991), AGPase promoter, B. Muller-Rober et al., Plant Cell 6: 601-612 (1994), proteinase inhibitor II promoter from potato (Keil, M. et al. (1989) EMBO J. 8: 1323-1330), or the cathepsin D inhibitor promoter from potato (Herbers, K. et al. (1994) Plant Mol. Biol. 26: 73-83). Also other tissue-specific promoters can be used like the fruit-specific promoter from tomato polygalacturonidase gene (Grierson, D. et al. (1986) Nucl.Acids Res. 14: 8595-8603), or the seed-specific phaseoline promoter from bean (Sengupta-Gopalan, C. (1985) Proc.Natl.Acad.Sci.USA 82: 3320-3324). Other promoters that can be used are inducible promoters, like the light inducible promoter derived from the pea rbcS gene (Coruzzi G. et al. (1984) EMBO J. 3: 1671-1679) and the actine promoter from rice (McElroy, D. et al. (1990) The Plant Cell 2: 163-171).

The promoters are to be found in the 5' region of each of the chimeric genes. At the 3' end of the promoter a short DNA sequence for 5' mRNA non-translated sequence may be added, which enhances translation of the mRNA transcribed from the chimeric genes. An example is the omega sequence derived from the coat protein gene of the tobacco mosaic virus (Gallie, D.R. et al. (1987) Nucl.Acids Res. 15: 3257-3273), or the alfalfa mosaic virus translational enhancer (Brederode, F.T. et al. (1980) Nucl.Acids Res. 8: 2213-2223).

Since the organelle in which lysine and most of the threonine biosynthesis takes place in higher plants is the plastid, both chimeric gene constructs comprise also each a DNA sequence coding for a transit peptide which is involved in the translocation of the protein from the cytosol into the plastids (Van den Broeck, G. et al. (1985) Nature 313: 358-363; Schreier, P.H. et al. (1985) EMBO J. 4: 25-32). This DNA sequence encoding a chloroplast transit peptide fused to a DNA sequence coding for AK or DHPS, will on expression produce a fused AK/transit peptide or DHPS/transit peptide chimeric protein in the cytoplasm of the transformed plant cell, which will be transported to the plastids, where increased production of both threonine and lysine is thereby obtained. DNA sequences coding for any kind of plastid transit peptide can be used in this invention, such as the DNA sequences derived from the ferredoxin gene coding for targeting of proteins into the chloroplast stroma (Smeekens, S. et al. (1985a) Nucl.Acids Res. 13: 3179-3194) and sequences from the plastocyanine gene coding for targeting of proteins into the chloroplast lumen (Smeekens, S. et al. (1985b) Nature 317: 456-458). The preferred DNA sequence coding for plastid targeting of AK and DHPS encodes the transit peptide originating from the pea rbcS-3A gene (Figure 2; Fluhr, R. et al. (1986) EMBO J. 5: 2063-2071).

The 3' end of the DNA sequence coding for the transit peptide is operationally fused to the DNA sequence encoding AK or DHPS, which is fused to a transcription termination DNA signal. This termination signal comprises a 3' transcription termination and a mRNA polyadenylation signal. Termination signals present at the 3' flanking region of any cloned gene can be used, e.g. from the pea rbcS gene, the bean phaseoline gene, or the nopaline synthase gene derived from the Ti plasmid of *Agrobacterium tumefaciens.* The preferred terminator sequence originates from the 3' flanking region of the octopine synthase gene from the Ti plasmid of *Agrobacterium tumefaciens* (Figure 2; Greve, H.D. et al. (1983) J.Mol.Appl.Genet. 1: 499-511).

The chimeric gene construct consists of two genes encoding AK and DHPS driven by different promoters, both present in the same binary vector (Figure 2). The AK encoding chimeric gene construct has the strong (enhanced) cauliflower mosaic virus 35S promoter in the 5' region linked to the 5' end of the omega DNA sequence; the omega DNA sequence is linked to the 5' end of the DNA sequence coding for the chloroplast transit peptide derived from the pea rbcS-3A gene, which is linked to the 5' end of the coding sequence of the mutant *E. coli* lysC allele coding for a desensitized AK-III, which is linked to the 5' end of the octopine synthase terminator sequence.

The DHPS encoding chimeric gene construct has basically the same structure, but the strong constitutive promoter of the AK gene construct is replaced by a weaker tuber-specific potato patatin (class I) promoter and the lysC gene coding for desensitized AK-III is replaced by the *E. coli* dapA gene coding for DHPS.

Both chimeric gene constructs can be subcloned into expression vectors, such as the Ti plasmids of *Agrobacterium tumefaciens,* the preferred plasmid being the binary vector pBINPLUS (Van Engelen, F.A. et al. (1995) Transgenic Research 4: 288-290).

### Selection of sink protein

For determining which protein can be used as a sink storage protein for essential amino acids a search is performed by searching the available entries in for example the SWISS-PROT and TrEMBL or NCBI GenBank databases. The first criteria for the protein is a high content of essential amino acids. Second criteria is the expected stability of the protein within the plant. Proteins with a natural storage function or a structural function within the original tissue are preferred. Alternatively, proteins that are known to form aggregates or regular crystal-like structures in the original tissue are selected. Third criteria is the absence of any unwanted activities interfering with health or growth of the plant as well as of the consuming animal (or man).

In addition to the expression constructs, as described above for the amino acid biosynthesis genes,the invention provides the use of expression constructs encoding the sink protein cDNA functionally linked at its 5'terminus to the translational enhancer sequence of omega, which is operationally linked at its 5'terminus to a strong promoter, being either the strong (enhanced) cauliflower mosaic virus 35S promoter for constitutive expression. Alternatively a strong tissue specific promoter, like GBSS, patatin, proteinase inhibitor II, cathepsin D etc. can be used. The 3' terminus of the sink protein cDNA is fused to a transcription termination DNA signal. This termination signal comprises a 3' transcription termination and a mRNA polyadenylation signal, or alternatively the 3' non coding sequence of the TMV virus, including its pseudoknot structure (Gallie, D.R. (1996) Plant Mol. Biol. 32:145-158). Termination signals present at the 3' flanking region of any cloned gene can be used, as described above for the DHPS or AK gene.

In addition to the expression construct for the sink protein an expression construct encoding the translational enhancer protein, like hsp101, is described. Also this gene is operationally flanked at its 5'terminus by a strong tissue specific promoter as described above. Alternatively, the strong (enhanced) cauliflower mosaic virus 35S promoter can be used for constitutive expression. The 3' terminus of the translational enhancer protein cDNA is fused to a transcription termination DNA signal, as described above.

The expression cassettes encoding for the different proteins, as described above, can be combined in one large gene construct, on one expression vector. Alternatively they can be combined in two or more expression vectors one for the amino acid biosynthesis genes, and another for the sink protein and/or the translational enhancer protein. Other combinations of two or three vectors containing different combinations of the described gene constructs are envisaged, that can be used to transform the plants in different order. The expression vector can for example be selected from the class of high copy number pUC or pBR322 derived plasmids, which will be used for direct transformation methods, like electroporation, particle bombardment or poly-ethylene-glycol mediated transformation procedures. Alternatively, the expression constructs can be included on a Ti plasmid derived, so-called binary vector, like pBINPLUS (Van Engelen, F.A. et al. (1995) Transgenic Research 4: 288-290). The Ti plasmids can be propagated in *Agrobacterium tumefaciens,* from which the inserted DNA fragments between the left and right border will be transferred to the plant cell. Other vectors are known in the art.

The expression vector in which a gene construct is cloned, is then introduced into cells. For plants, the introduction can be realised using any kind of transformation protocol capable of transferring DNA to either monocotyledonous or dicotyledonous plant cells. For example: transformation of plant cells by direct DNA transfer via electroporation (Dekeyser, R.A. et al. (1990) The Plant Cell 2: 591-602), via PEG precipitation (Hayashimoto, A. et al. (1990) Plant Physiol. 93: 857-863) or via particle bombardment (Gordon-Kann, W.J. et al. (1990) The Plant Cell 2: 603-618), and DNA transfer to plant cells via infection with Agrobacterium. The preferred method is via infection of plant cells with *Agrobacterium tumefaciens* (Horsch, R.B. et al. (1985) Science 227: 1229-1231; Visser, R.G.F. (1991) Plant Tissue Culture Manual B5 (ed. by K. Lindsey): 1-9, Kluwer Acad. Publishers, The Netherlands). The methodology used here for potato also can be used to improve crop plants like sugar beet, carrot, cassave, canola, alfalfa, legumes, and gramineae species like rice, maize, wheat, sorghum, barley and grasses like *Lolium perenne.*

Transformed plants are then selected by resistance to kanamycin or other antibiotics like hygromycin, or herbicides like bialaphos or phosphinotricin or by using selection markers such as xylose isomerase or phosphomannose isomerase. Selection for plant cells or plants that overproduce amino acids such as tryptophan, methionine, threonine and lysine or amino acid analogues may also be performed by adding tryptophan, methionine, threonine or lysine to the plant growth media.

Plants containing in their cells for example the chimeric AK and DHPS gene constructs as well as the gene constructs for the sink protein and the translational enhancer, like hsp101, can also be obtained by crossing (or retransforming) a transgenic plant containing only a subset of the described gene constructs with a transgenic plant containing the complementing subset of gene constructs.

As an example the use of a combination of gene constructs containing a DNA sequence encoding an enzyme having aspartate kinase (AK) activity and a DNA sequence encoding an enzyme having dihydrodipicolinate synthase (DHPS) activity combined with a DNA sequence encoding a sink protein that is rich in essential amino acids, e.g. vicilin, and a DNA sequence encoding a protein factor involved in specifically enhancing the translation of the sink protein is provided. First half of this construct containing the AK and DHPS genes is capable of differential expression of the two genes resulting in an increased level of both free lysine and threonine more than 5-fold the wild type level of each amino acid in a plant or parts thereof. This expression is now possible without incurring the previous problems associated with high accumulation of lysine or combined expression of both AK and DHPS genes in a plant. The expression regulation should be such that expression occurs in such a way that both lysine and threonine are produced to a comparable extent without damaging the plant i.e. without causing negative aberrations in the phenotype compared to wild type plants. The second part of this combination of gene constructs consists of a gene encoding a sink protein, which contains a high amount of essential amino acids. This sink protein results in an increased incorporation of essential amino acids into the protein fraction. As such it withdraws these amino acids from the pool of free amino acids, thus further enhancing the synthesis of these essential amino acids. The synthesis of this sink protein on its turn is enhanced by a protein factor that specifically recognises a sequence domain present in the sink protein's messenger RNA. The gene encoding this protein factor is also part of this combination of gene constructs and introduced into the plant. The examples and combinations thereof describe methods and combinations thereof which enhance both the synthesis of essential amino acids in plant cells, and enhances the protein content as well as the protein composition in the transformed plant, a new approach to improve the feed quality of crops.

### Example 1. Construction of the chimeric double gene construct for expression of AK and DHPS

DNA isolation, subcloning, restriction analysis and DNA sequence analysis is performed using standard methods (Sambrook, J. et al. (1989) Molecular Cloning. A laboratory manual, Cold Spring Harbor Laboratory Press; Ausubel, F.M. et al. (1994) Current protocols in molecular biology, John Wiley & Sons).

The chimeric gene containing the lysC gene is constructed by fusing the enhanced CaMV35S promoter to the chimeric lysC gene (Shaul, O. and Galili, G (1992) Plant Physiol. 100: 1157-1163). This chimeric lysC construct contains the DNA fragment of the mutant lysC allele which is fused at the 5' end to the omega DNA sequence from the coat protein of tobacco mosaic virus (Gallie, D.R. et al. (1987) Nucl.Acids Res. 15: 3257-3273). Downstream of the lysC sequences the termination signal of the octopine synthase gene from Agrobacterium tumefaciens is inserted (Greve, H.D. et al. (1983) J.Mol.Appl.Genet. 1: 499-511). A DNA fragment containing the pea rbcS-3A transit peptide coding sequence (Fluhr, R. et al. (1986) EMBO J. 5: 2063-2071) is inserted between the A DNA and the lysC coding sequence. The chimeric lysC gene construct is cloned as a BamHI/SpeI fragment in pBluescript (Shaul, O. and Galili, G (1992) Plant Physiol. 100: 1157-1163). The enhanced CaMV35S promoter is ligated as a SmaI/BamHI fragment in front of the lysC chimeric gene digested with BamHI/XbaI in the XbaI/SmaI fragment of the binary vector pBINPLUS (Van Engelen, F.A. et al. (1995) Transgenic Research 4: 288-290) (pAAP30; Figure 2A).

The chimeric gene containing the DapA gene is constructed by fusing the enhanced CaMV35S promoter to the chimeric DapA gene (Shaul, O. and Galili, G. (1992) Plant J. 2: 203-209). This chimeric DapA construct contains the DNA fragment of the *E. coli* DapA gene which is fused at the 5' end to the omega DNA sequence from the coat protein of tobacco mosaic virus (Gallie, D.R. et al. (1987) Nucl.Acids Res. 15: 3257-3273). Downstream of the lysC sequences the termination signal of the octopine synthase gene from Agrobacterium tumefaciens is inserted (Greve, H.D. et al. (1983) J.Mol.Appl.Genet. 1: 499-511). A DNA fragment containing the pea rbcS-3A transit peptide coding sequence (Fluhr, R. et al. (1986) EMBO J. 5: 2063-2071) is inserted between the omega DNA and the lysC coding sequence. The chimeric DapA gene construct is cloned as a BamHI/SpeI fragment in pBluescript (Shaul, O. and Galili, G. (1992) Plant J. 2: 203-209). The patatin promoter (Wenzler, H.C. et al. (1989) Plant Mol.Biol. 12: 41-50) is ligated as a blunt (HindIII filled in)/BamHI fragment in front of the DapA chimeric gene digested with SmaI/BamHI (pAAP31; Figure 2B).

The double gene construct is realised by ligating the enh.CaMV-35S-dapA chimeric gene as a KpnI/SacI fragment from pAAP31 into the binary vector with patatin-DapA (pAAp30) digested with SacI/KpnI (pAAP50; Figure 2C).

### Example 2. Introduction of the chimeric genes into potato

### 2.1 Transformation of potato plants

The binary vector pAAP50 is used for freeze-thaw transformation of *Agrobacterium tumefaciens* strain AGLO (Höfgen, R. and Willmitzer, L. (1988) Nucl.Acids Res. 16: 9877). Transformed AGLO is subsequently used for inoculation of diploid potato *(Solanum tuberosum,* variety Kardal) stem explants as described by Visser (Visser, R.G.F. (1991) Plant Tissue Culture Manual B5 (ed. by K. Lindsey): 1-9, Kluwer Acad. Publishers, The Netherlands). After shoot and root regeneration on kanamycin-containing media plants are put in soil and transferred to the greenhouse. Plants regenerated (on kanamycin-free media) from stem explants treated with the Agrobacterium strain AGLO lacking a binary vector serve as a control.

### 2.2 In vitro tuber formation

In order to induce in vitro tuberization, nodal cuttings (about 4-5 cm long) of transformed potato plantlets are placed vertically in solid Murashige and Skoog medium (Murashige, T. and Skoog, F. (1962) Physiol.Plant. 15: 473-497) supplemented with 10% (w/v) sucrose, 5 TM BAP. The cultures are maintained at 19°C in the dark. After 14 days microtubers of 4 mm in diameter are harvested and analysed for free lysine and threonine content, and for AK and DHPS activity. The protein content and the protein composition are analysed as described below.

### Example 3. Application of the invention in perennial ryegrass (Lolium perenne L.)

*Plant materials -* Embryogenic suspension cultures from different *Lolium perenne* cultivars, a.o. the cultivars Moronda and Aurora, are initiated directly from mature, seed-derived embryos or from embryogenic callus cultures obtained from immature inflorescence segments, essentially as described by Creemers-Molenaar et al., *Plant Science* 63:167-176 (1989). For the direct approach, seeds are sterilized in 10% hypochlorite, rinsed and soaked for two days in sterile tap water and sterilized for a second time. After rinsing thoroughly, mature embryos are dissected from 40 seeds, chopped and transferred to 5 ml of MS10 medium (i.e. Murashige and Skoog basal salts and vitamins supplemented with 10 mg/l 2,4-D and 3% sucrose at pH 5.8) in a 60 ml plastic specimen container (Thovadex). This in several replicates. Embryogenic callus is induced on immature inflorescence segments of greenhouse grown plants after sterilization with 5% hypochlorite and rinsing with sterile water. The basal parts of the inflorescences are cut into 2 segments of 1-2 mm long and placed on MSt5 medium (i.e. MS basal salts and vitamins supplemented with 0.4 mg/l (=extra) thiamin-HCl, 5 mg/l 2,4-D and 3% sucrose) solidified with 0.8% Daichin agar. Culture is in the dark at 25 °C. After 4-8 weeks compact, embryogenic callus is excised from the explants of 2-5 plants (i.e. genotypes). The calli are mixed, chopped with a scalpel and transferred in 0.1 g FW aliquots to 5 ml of MS10 in specimen containers. From this point on, the cultures of both origins are treated identically. The cultures are incubated on a rotary shaker (140 revs./min.) in continuous indirect light (200-400 lux) at 25 °C. After 10 days the medium is replaced with MS5 ( = as MS10 but with 5 mg/l 2,4-D). Once a week 2 ml of fresh medium is added to the cultures until a final volume of 15 ml is reached. Subsequently, the cultures are transferred to new 190 ml transparent, polystyrene containers (Greiner) and 5 ml fresh medium is added. For further experimentation well-proliferating, finely-dispersed cultures are selected and maintained by weekly subculturing 2.5 g FW material in 20 ml fresh MS5. Incubation is in the dark under continuous shaking at 120 revs./min. at 25 °C.

The regeneration potential of the cultures is determined by placing 0.5-1.0 g FW material on solid MS0 (0 mg/l 2,4-D; 0.8% agar). After culturing in the dark at 25 °C for the first 2 weeks the calli are placed in dimmed light (500-1000 lux) for 16 hours/day for the next 2 weeks. Finally after transfer to fresh MS0, they are placed in 4000 lux, 16 hours/day and the number of calli producing shoots is scored after 4 weeks.

*Transformation -* Three days after subculture, 0.25 g FW callus material consisting of cells in log-phase, is evenly dispersed onto the surface of a 42 mm diameter Whatman filter disc (Schleicher & Schuell #604). Subsequently, the filters are moistened by the addition of 0.5 ml fresh culture medium and they are placed onto culture medium solidified with 0.2% gelrite and left overnight at 25 °C in the dark. The next day the filters carrying the perennial ryegrass suspension material are used for biolistic gene transfer using the PDS1000-He particle gun (BioRad). 0.375 mg Gold particles with an average diameter of 1 µm were coated with 0.625 µg DNA of plasmid PAAP60, which is a derivative of the plasmid pABC mentioned in Example 1 lacking T-DNA borders and with the *npt*II gene replaced by the hpt gene driven by the CaMV35S promotor for selection of transgenic perennial ryegrass cells. For the coating, 50 µl (= 3 mg) of washed particles are suspended thoroughly by vortexing. Subsequently, 5 µl plasmid DNA (concentration 1 µg/µl) and 50 µl 2.5M CaCl₂ are added and vortexed for 10 seconds. Then, 20 µl 0.1M free-base spermidine is added and mixed by vortexing for 2 seconds. The mixture is centrifuged for 5 seconds and the supernatant is removed, after which 250 µl ethanol 96% is added followed by vortexing for 1 minute. After washing with ethanol once, the particles now coated with DNA are resuspended in 60 µl ethanol 96% and kept on ice until use. Bombardment can be performed at pressures ranging from 1100 psi to 2200 psi, but in this example particularly a pressure of 1800 psi is used while the dish containing the filters is placed at a distance of 9 cm. After biolistics the filters are incubated on the culture medium in the dark at 25 °C for 24 hours before they are transferred to selection medium. For selection, the filters are first placed on culture medium MSt5 solidified with 0.2% gelrite containing 80 mg/l hygromycin; after 1 week the filters are transferred to selection medium containing 150 mg/l hygromycin. So far, this is essentially as described earlier (Van der Maas et al., *Plant Mol. Biol.* 24:401-405 [1994]). Actively growing calli are individually transferred to fresh selection medium (150 Hyg.) after 4 weeks. Following this second round of selection surviving calli are placed on regeneration medium supplemented with 50 mg/l hygromycin. Transgenic perennial ryegrass plants are obtained and collected after 8 weeks. They are maintained in tubes containing half strength MS0 (Creemers-Molenaar et al., *Plant Science* 57: 165-172 [1988]) and subsequently characterized molecularly by PCR and Southern hybridization analysis and biochemically by amino acid , enzyme activity and protein analysis as described in Example 4 - 9 to confirm presence and expression of the newly introduced genes.

### Example 4. Analysis of free amino acid content in transgenic plants

Tissue (0.5-1.0 gram) is homogenized with mortar and pestle in 2 ml 50 mM Pi-buffer (pH 7.0) containing 1 mM dithiothreitol. Nor-leucine is added as an internal standard. Free amino acids are partly purified by extraction with 5 ml of a water:chloroform:methanol mixture (3:5:12). Water phase is collected and the remaining re-extracted twice. After concentration by lyophilization to 3 ml, a 20 µl sample is analysed by HPLC using a cation-exchange column with post-column ninhydrine derivatisation of the amino acids detected at 570 and 440 nm (BIOCHROM 20, Amersham Pharmacia biotech).

### Example 5. Analysis of key enzyme activity of essential amino acid biosynthesis pathways

Enzyme activity of plant material can be analysed by measuring decrease or increase in absorbency at specific wave lengths based on disappearance of the substrate or appearance of the product of the enzyme activity. This can also be the conversion of NAD(P)H into NAD(P). Examples of analysis methods of AK and DHPS activity are given below in example 6 and 7.

### Example 6. Analysis of AK activity in plants

Microtubers, leaves or mature tubers are harvested and homogenized with mortar and pestle in an equal volume of cold 20 mM potassium-phosphate buffer, pH 7.0, containing 30 mM β-mercaptoethanol, 0.1 mM L-lysine, 0.1 mM L-threonine, 1 mM phenylmethylsulfonylfluoride and 0.5 Tg/ml leupeptin. Following 5 min of centrifugation (16,000 g, 4°C), the supernatant is collected and its protein concentration determined. Equal amounts of protein are then tested for AK activity as described by Black et al. (Black, S. et al. (1955) J.Biol.Chem. 213: 27-38). The assay mixture (final volume of 0.25 ml) contained 300 µg of protein, 100 mM Tris-HCl, pH 8.0, 200 mM NH₂OH, neutralised prior to use with KOH, 10mM ATP, 5mM MgCl, and 30 mM L-aspartate. The control assays contains all the ingredients except L-aspartate. The reactions are performed at 37°C for 1 h and then terminated by addition of 0.25 ml of a 1:1:1 solution of 10% (w/v) FeCl3 in 0.1 N HCl, 3 N HCl and 12% TCA mixed prior to use. Following 5 min on ice, the reaction mixture is centrifuged (5 min 16,000 g, 4°C) and the light absorbency of the supernatant at 490 nm is measured. For each sample, the nonspecific activity obtained in the absence of L-aspartate is substracted from that obtained in its presence.

### Example 7. Analysis of DHPS activity in plants

Microtubers or mature tubers are homogenized with mortar and pestle in an equal volume of cold 100mM Tris-HCl pH 7.5 containing 2 mM EDTA, 1.4% sodium ascorbate, 1mM phenylmethylsulphonilfluoride and 0.5 Tg/ml leupeptin. Following 5 min. centrifugation (16,000 g at 4°C) the supernatant is collected. DHPS activity was measured using the O-aminobenzaldehyde (O-ABA) method of Yugari and Gilvarg (Yugari, Y. and Gilvarg, C. (1965) J.Biol.Chem. 240: 4710-4716).

### Example 8. Protein analysis of plants

The plant materials are frozen in liquid nitrogen and stored at -80°C. The frozen material is homogenized with mortar and pestle, while being cooled with liquid nitrogen. The resulting powder is weighed and subsequently extracted three times with a cold 80% aceton/water mixture and separated by centrifugation. The resulting pellet fraction is solubilised in 6M urea, 2M thiourea, 10mM DTT ( or 2mM TBP), 40mM Tris. Alternatively the frozen plant powder is directly solubilised in SDS-Sample buffer, according to Laemmli (125mM Tris/HCl, 4% SDS, 20% glycerol, 50mM DTT, 0.02% BromophenolBlue, pH=6.8 ). Total protein concentration is analysed using Bradford staining method (Ausubel, F.M. et al. (1994) Current protocols in molecular biology, John Wiley & Sons), applied directly on the urea soliubilised extract or on an aceton precipitate from the Laemmli solubilised sample. The relative CBB G250-stain-binding property of the extracted sample is compared to a series of reference samples containing increasing amount of bovine serum albumin as standard.

The protein composition of the extracted sample is analysed by separation using either one-dimensional SDS-PAGE according to Laemmli, U.K. Nature (1970) V227, 680-685, or in more detail using two-dimensional separation essentially as described in Görg, A. et.al, Electrophoresis (1985) V6,599-604 and Dunn,M.J., Corbett,J.M. Meth. Enzymol. (1996) V271, 177-203.

### Example 9. Analysis of protein bound amino acid content in plants

Plant tissue (0.2-0.5 mg) is homogenized with mortar and pestle in liquid nitrogen. Ground tissue is extracted three times with cold 80% aceton/water mixture and separated by centrifugation. Amino acid content in the protein fraction is determined after acid hydrolysis according to Cohen and De Antonis (Cohen and De Antonis (1994) J. of Chromatography 661: 25-34), or (for tryptophan and cysteine) according to Manneberg et al. (Manneberg, M et al. (1995) Anal. Biochem. 224: 122-127).

### REFERENCES

- Ausubel, F.M. et al. (1994) Current protocols in molecular biology, John Wiley & Sons
- Benfey, P.N. et al. (1990) EMBO J. 9: 1685-1696.
- Black, S. et al. (1955) J.Biol.Chem. 213: 27-38.
- Brederode, F.T. et al. (1980) Nucl.Acids Res. 8: 2213-2223.
- Bright,S.W.J. et al. (1982) Nature 299: 278-279.
- Bryan, J.K. (1980), The Biochemistry of Plants (ed. by B.J. Miflin) Vol. 5: 403-452, Academic Press, N.Y.
- Casan, M. et al. (1986), J.Biol.Chem. 261: 1052-1057.
- Cattoir-Reynaerts A. et al. (1983) Biochem.Physiol.Pflanzen 178: 81-90.
- Cohen and De Antonis (1994) J. of Chromatography 661: 25-34
- Coruzzi G. et al. (1984) EMBO J. 3: 1671-1679.
- Dekeyser, R.A. et al. (1990) The Plant Cell 2: 591-602
- Dotson, S.B. et al. (1990) Planta 182: 546-552.
- Dunn,M.J., Corbett,J.M. Meth. Enzymol. (1996) V271, 177-203
- Falco,S.C. et al. (1995), Bio/Technology 13: 577-582.
- Fluhr, R. et al. (1986) EMBO J. 5: 2063-2071.
- Frankard, V. et al. (1992) Plant Physiol. 99: 1285-1293.
- Galili, G. (1995) The Plant Cell 7: 899-906.
- Gallie, D.R. et al. (1987) Nucl.Acids Res. 15: 3257-3273
- Gallie, D.R. (1996) Plant Mol. Biol. 32:145-158).
- Glassman, K.F. (1992) Biosynthesis and Mol. Regul. of Amino Acids in Plants (ed. by B.K. Singh et al.): 217-228.
- Gorg, A. et.al, (1985) Electrophoresis V6,599-604
- Gordon-Kann, W.J. et al. (1990) The Plant Cell 2: 603-618.
- Greve, H.D. et al. (1983) J.Mol.Appl.Genet. 1: 499-511.
- Grierson, D. et al. (1986) Nucl.Acids Res. 14: 8595-8603.
- Hayashimoto, A. et al. (1990) Plant Physiol. 93: 857-863.
- Herbers, K. et al. (1994) Plant Mol. Biol. 26: 73-83.
- Höfgen, R. and Willmitzer, L. (1988) Nucl.Acids Res. 16: 9877.
- Horsch, R.B. et al. (1985) Science 227: 1229-1231.
- Keil, M. et al. (1989) EMBO J. 8: 1323-1330).
- Laemmli, U.K. (1970) Nature V227, 680-685 Lea, P.J. et al. (1985) Chemistry and Biochemistry of the Amino Acids (ed. by G.C. Barrett): 197-226, London: Chapman and Hall.
- Manneberg, M et al. (1995) Anal. Biochem. 224: 122-127
- Matthews, B.F. et al. (1989) Plant Physiol. 91: 1569-1574.
- McElroy, D. et al. (1990) The Plant Cell 2: 163-171.
- Mignery, C.A. et al. (1988) Gene 62: 27-44.
- Murashige, T. and Skoog, F. (1962) Physiol.Plant. 15: 473-497.
- Negrutiu, I. et al. (1984) Theor.Appl.Genet. 6: 11-20.
- Pen, J. et al. (1992) Bio/Techn. 10: 292-296.
- Perl, A. et al. (1992) Plant Mol.Biol. 19: 815-823.
- Richaud, C. et al.( 1973) Eur.J.Biochem. 40: 619-629.
- Richaud, F. et al. (1986) J.Bacteriol. 166: 297-300.
- Sambrook, J. et al. (1989) Molecular Cloning. A laboratory manual, Cold Spring Harbor Laboratory Press.
- Schreier, P.H. et al. (1985) EMBO J. 4: 25-32.
- Sengupta-Gopalan, C. (1985) Proc.Natl.Acad.Sci.USA 82: 3320-3324.
- Shaul, O. and Galili, G. (1992a) Plant J. 2: 203-209.
- Shaul, O. and Galili, G. (1992b) Plant Physiol. 100: 1157-1163.
- Shaul, O. and Galili, G. (1993) Plant Mol. Biol. 23: 759-768.
- Shedlarski, J.G. and Gilvarg, C. (1970) J.Biol.Chem. 245: 1362-1373.
- Smeekens, S. et al. (1985a) Nucl.Acids Res. 13: 3179-3194.
- Smeekens, S. et al. (1985b) Nature 317: 456-458.
- Umbarger, H.E. (1978) Ann.Rev.Biochem. 47: 533-606.
- Van den Broeck, G. et al. (1985) Nature 313: 358-363.
- Van Engelen, F.A. et al. (1995) Transgenic Research 4: 288-290.
- Van Gelder,W.M.J. and Vonk,C.R. (1980) Potato Res. V23 427-434
- Visser, R.G.F. (1991) Plant Tissue Culture Manual B5 (ed. by K. Lindsey): 1-9, Kluwer Acad. Publishers, The Netherlands.
- Wenzler, H.C. et al. (1989) Plant Mol.Biol. 12: 41-50.
- Yugari, Y. and Gilvarg, C. (1962) Biochem.Biophys.Acta 62: 612-614.
   Yugari, Y. and Gilvarg, C. (1965) J.Biol.Chem. 240: 4710-4716.

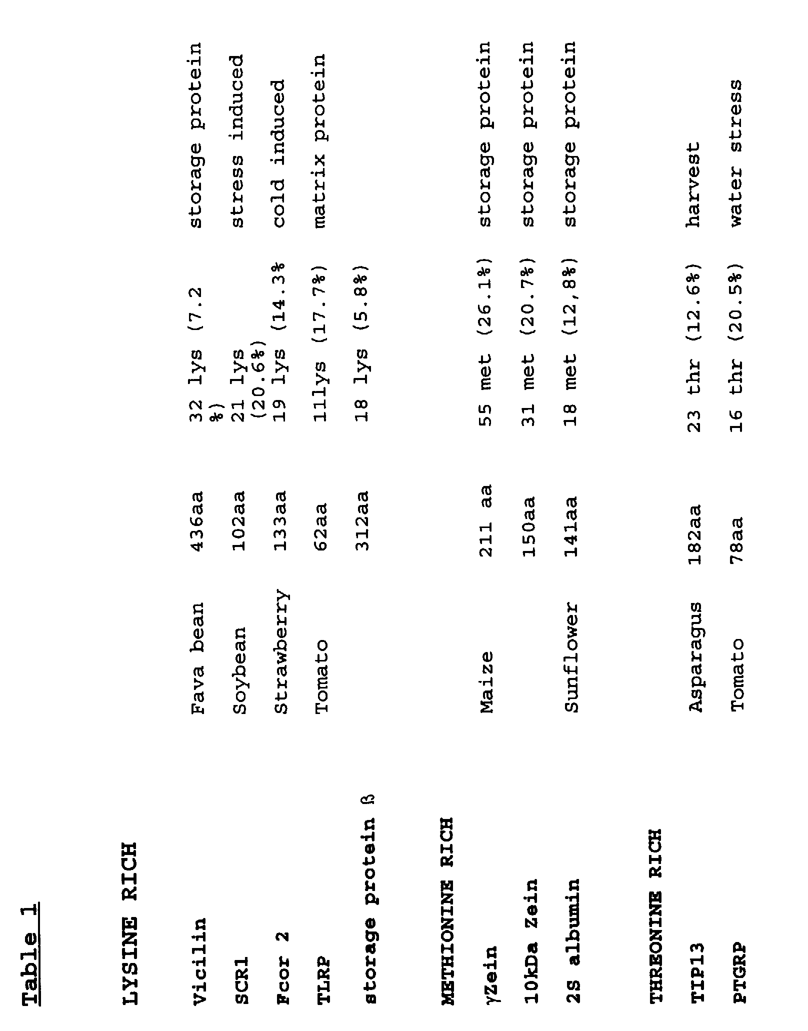

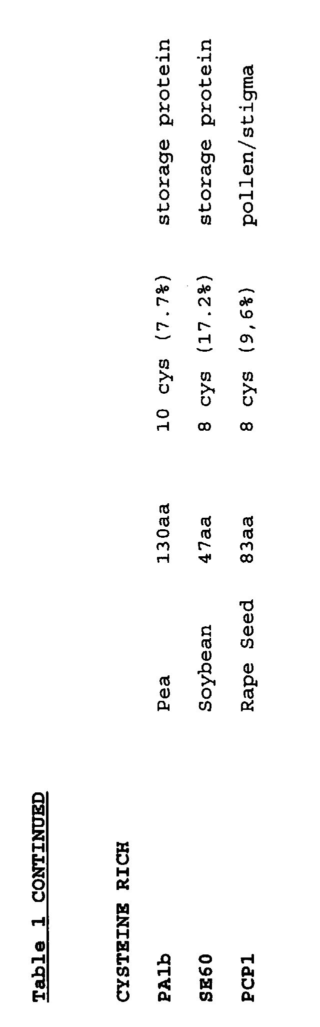

## Claims

1. A method for regulating supply of a desired amino acid in a cell comprising providing said cell with a nucleic acid encoding a sink protein comprising at least said amino acid thereby creating a demand in said cell for said amino acid.

2. A method according to claim 1 further comprising providing said cell with a nucleic acid allowing enhanced translation of said sink protein.

3. A method according to claim 1 or 2 further comprising providing said cell with a nucleic acid encoding at least one functional enzyme related to said amino acid's biosynthesis pathway allowing said cell to synthesise said amino acid.

4. A method according to anyone of claims 1 to 3 wherein said amino acid is an essential amino acid.

5. A method according to claim 4 wherein said amino acid is tryptophan, methionine, threonine or lysine.

6. A method according to anyone of claims 1 to 5 wherein said cell is a plant cell.

7. A transgenic cell having been provided with a nucleic acid encoding a sink protein comprising at least a desired amino acid creating a demand in said cell for said amino acid allowing regulating the concentration of said amino acid in said cell.

8. A cell according to claim 7 further having been provided with a nucleic acid allowing enhanced translation of said sink protein.

9. A cell according to claim 7 or 8 further having been provided with a nucleic acid encoding at least one functional enzyme related to said amino acid's biosynthesis pathway allowing said cell to synthesise said amino acid.

10. A cell according to anyone of claims 7 to 9 wherein said amino acid is an essential amino acid.

11. An organism derived from a cell according to anyone of claims 7 to 10.

12. An organism according to claim 11 which is a plant.

13. Foodstuff for an animal comprising at least part of a cell according to anyone of claims 7 to 9 or of an organism according to claims 11 or 12.

14. Foodstuff according to claim 13 wherein said animal is monogastric.

15. A food animal product derived from an animal fed a foodstuff according to claims 13 or 14.

16. Use of at least part of a cell according to anyone of claims 7 to 9 or of an organism according to claims 11 or 12 for increasing nutritional value of a foodstuff.
